# EUROPEAN PATENT SPECIFICATION

(11) **EP 1 945 278 B1**
(45) Date of publication and mention of the grant of the patent: **12.05.2010**
(21) Application number: 07804732.1
(22) Date of filing: 31.07.2007
(51) Int. Cl.: A61L 9/04, A61L 9/12, F24F 3/16

(54) **AN ELECTRIC DOMESTIC APPLIANCE COMPRISING A KIT AND A METHOD FOR PRODUCING THE KIT**
HAUSHALTSGERÄT MIT EINEM BAUSATZ UND VERFAHREN ZUR ERZEUGUNG DES BAUSATZES
APPAREIL MÉNAGER ÉLECTRIQUE COMPRENANT UN KIT ET PROCÉDÉ DE PRODUCTION DU KIT

(30) Priority: 20.11.2006 IT RN20060074
(43) Date of publication of application: 23.07.2008
(73) Proprietor: Indesit Company S.p.A., 60044 Fabriano (AN) (IT)
(72) Inventor: POLVERINI, Davide, 60126 Ancona (IT); FARALDI, Paolo, 18038 San Remo (Imperia) (IT); CORRIAS, Silvio, 14036 Moncalvo (Asti) (IT)
(74) Representative: Paolizzi, Marco
(86) International application number: PCT/IB2007/002278
(87) International publication number: WO 2008/062260

(56) References cited:
- WO-A-01/56622
- WO-A-02/42364
- JP-A- 2005 324 488
- US-A1- 2002 058 595

## Description

### Technical Field

This invention relates to an electric domestic appliance comprising a kit and a method for producing this kit for an electric domestic appliance.

### Background Art

Document US2002/058595 discloses a device for deodorizing an environment comprising perfumed particles emitting their perfume outside the container. Document WO01/56622 discloses an apparatus for deodorizing air in confined spaces comprising a first and a second filter member which are attachable to a moving member. Document WO02/42364 discloses a method for producing a polyethylene or polypropylene plastic film having an odor barrier material incorporated into said film.

Electric domestic appliances are known which comprise at least one internal compartment, kept closed during normal functioning by an access door, to which a seal is usually attached to ensure airtight closure of the internal compartment. These appliances include, for example, refrigeration appliances and washing appliances: in refrigeration appliances the internal compartment consists of a refrigerator or a freezer, in which foodstuffs are placed to be cooled or frozen, while in washing appliances the internal compartment consists of a drum or chamber in which items of clothing (if the appliance is a washing machine) or dishes (if the appliance is a dishwasher) are placed in order to be washed.

An electric domestic appliance is traditionally packaged at the end of the production cycle: this means that the relative internal compartment remains closed until the appliance is removed from the packaging and positioned in the home of the user, or displayed in a store in order to be seen, and possibly chosen, by a customer.

It should be pointed out that a fairly significant period of time may pass, even several months, between the time of packaging and the subsequent opening of the door which keeps the internal compartment closed.

Once the packaging has been removed, known electric domestic appliances traditionally present the problem of emitting an unpleasant odour when the door providing access to the internal compartment is opened. This is a fairly serious problem. In fact, when the appliance is on display in a store, the customer may, on smelling this unpleasant odour, consciously or unconsciously associate this appliance with a poor level of quality and be persuaded to purchase a competitor's appliance. The unpleasant odour does in fact have an immediate negative impact on the customer. In the case of a refrigerator, the unpleasant odour is due essentially to the release of monomers by the polymers forming the inner walls of the refrigerator or freezer, these polymers being in particular styrene-based polymers (mainly polystyrene or ABS). The continuous release of monomers, in the period during which the appliance remains in its packaging, means that when the door is opened the first few times there is a characteristic and fairly intense odour which is commonly associated with plastic.

The cause of the unpleasant odour in the internal compartment of a washing appliance is different: in this case, the unpleasant odour is due to the fungi, mould and bacteria which develop from the few drops of water present in the drum, these being the remains of the water used during the testing cycle carried out before the appliance was sealed and packed.

To counter the phenomenon of the unpleasant odour that accumulates in the internal compartment of an electric domestic appliance, in particular a refrigeration or washing appliance, when it is packaged, a series of possible solutions have been devised and implemented:
i) a first solution consists of spraying a scented spray in the internal compartment of the appliance or of placing a solid or liquid scented essence in it. This operation must be carried out before the appliance is packaged. This solution presents the disadvantage of requiring an additional operation in the production cycle of the appliance, with the inevitable increase in cost, time and complexity of the production process;
ii) a second solution consists of applying a coating which comprises a scented essence to the surface of the walls of the internal compartment of the appliance or to a portion of this surface. As well as requiring an additional operation, this solution leads to the serious problem that the scent remains during the functioning of the appliance: this is particularly unacceptable if the appliance is a refrigeration appliance, since this scent alters the smell of the food contained inside the appliance;
iii) a third solution, specifically for refrigeration appliances, consists of adding a scented essence to the plastic used to form the inner walls of the refrigerator or freezer. In addition to the impossibility of removing the scent before the appliance is put into operation, this solution presents the problem that most of the scented essence is ineffective and thus wasted. In fact, the walls of a refrigerator or freezer are generally between 0.8 mm and 1.5 mm thick, but only the scented essence on the surface of these walls and which comes into contact with the air in the internal compartment is effective against the unpleasant odour due to the release of monomers.

### Disclosure of the Invention

The aim of this invention is to resolve the problems described above, providing a kit with electric domestic appliances that counters the unpleasant odours generated in the internal compartment of an electric domestic appliance during the period starting with the airtight closure of the internal compartment prior to packaging up to the opening of the door after removal of the packaging.

Another aim of the invention is to provide a kit with electric domestic appliances that can carry out a sanitising action on the micro-organisms that are present in this internal compartment.

A further aim is to counter these unpleasant odours and/or presence of undesired micro-organisms without foreseeing any additional working operation during the production cycle of the appliance and without requiring any additional operation on the part of the user with respect to what is usually carried out between the purchase and start up of the appliance.

Additional aims of this invention are to provide an electric domestic appliance comprising the above-mentioned kit and a procedure for the production of the above-mentioned kit.

These and other aims are achieved by means of an electric domestic appliance comprising the above-mentioned kit and a procedure for the production of the above-mentioned kit with the characteristics disclosed in the appended claims which represent an integral part of this description.

The general idea on which this invention is based is to counter the unpleasant odours generated in the internal compartment of an electric domestic appliance, providing deodorising and/or aromatising and/or sanitising properties to a kit for electric domestic appliances.

### Detailed Description of the Preferred Embodiments of the Invention

The subject of this invention is therefore a kit with an electric domestic appliance which kit comprises:
i) a first component consisting of paper documentation relative to the appliance, for example an instruction manual and/or the guarantee of the appliance;
ii) a second component consisting of a holder that defines an internal cavity in which the paper documentation is contained.

According to this invention, at least one of these first and second components emits particles able to counter the unpleasant odours of the surrounding outer environment.

In particular, the particles able to counter the unpleasant odours of the surrounding outer environment are emitted by the holder.

In a preferred embodiment, the holder of the kit according to this invention differs from the bags at present normally used to contain and protect the instruction manuals of electric domestic appliances since it is provided with a variety of functions, namely that of pleasantly scenting the internal compartments of appliances, in particular refrigeration and washing appliances, and/or of countering and/or limiting and/or destroying the unpleasant odours and/or micro-organisms and/or pathogenic agents that are generated in this compartment.

Advantageously, the holder is flexible. The holder preferably comprises a bag and even more preferably consists of a bag.

The particles preferably perform a deodorising action by means of which they tend to eliminate any odours in the surrounding external environment.

The particles advantageously perform an aromatising action by means of which they tend to cover the odours in the surrounding external environment with their scent. The majority of the particles emitted by the scented essences carry out a combined deodorising and aromatising action.

The instruction manual of the kit according to this invention can be any one of those currently used by the manufacturers of appliances to provide users with information such as: installation method, maintenance, technical data of the product, etc.

The holder is preferably made from polymeric material, even more preferably from ethylene polymers and/or copolymers, in particular from polyethylene (for example low density polyethylene, known commercially as LDPE) or from ethylene vinyl acetate (EVA).

The material of the holder advantageously comprises a scented essence that emits particles with an aromatising and/or deodorising action.

In particular, the particles comprise the volatile molecules emitted by the scented essences. The scented essence is selected on the basis of the desired aroma to be provided in the internal compartment of the appliance: among the possible aromas, fruit aromas (lemon, strawberry, green apple, etc.) appear to be particularly suitable for this invention. To provide a strawberry aroma, for example, one of the essential volatile compounds of the strawberry can be used, for example one of the 91 esters identified by Tressl in 1969 (1-pentyl acetate, 1-pentyl n-butyrate, 1-pentyl n-caproate, etc.).

The quantity of scented essence in the holder can vary considerably depending on the type of scented essence and/or the volume of the internal compartment in which the kit must be placed and/or the desired concentration of particles of scented essence to be released in the internal compartment of the appliance. The scented essence preferably constitutes from 1% to 20% of the weight of the holder, even more preferably from 8% to 12% of the weight of the holder. The scented essence advantageously constitutes 10% of the weight of the holder.

The thickness of the holder is extremely limited, preferably between 0.01 mm and 0.5 mm, even more preferably between 0.1 mm and 0.3 mm, advantageously 0.2 mm. The limited thickness means that a significant amount of scented essence is effective because it is in direct contact with the air in the internal compartment of the appliance.

In a preferred embodiment, the scented essence is uniformly distributed in the holder. Alternatively, the holder comprises an inner layer and an outer surface layer chemically different from the inner layer, the particles being emitted by the outer surface layer. The inner layer and the outer surface layer are advantageously closely adherent to each other. The outer surface layer is preferably applied to the inner layer by means of painting.

The particles emitted also perform a sanitising action. In this case, for example, the particles comprise silver ions previously applied to the holder and gradually released.

The sanitising action is important in order to eliminate micro-organisms (fungi, mould, bacteria, etc.) that are responsible for unpleasant odours. This is particularly evident in washing appliances which may contain residual traces of water from the testing cycle carried out before the appliance is sealed and packaged.

In an alternative embodiment, part of the paper documentation emits the particles with a deodorising and/or aromatising and/or sanitising action. In this case, the holder is permeable to the particles tp allow the particles to pass through the holder from the inside to the outside. In particular, the holder can present holes. Advantageously, the documentation paper is scented and the particles are scented essence particles emitted by the paper. The particles can also have a sanitising action as, for example, in the case of silver ions. In this case, the ions are previously applied to the paper documentation and then gradually released.

Subject of this invention is an electric domestic appliance comprising:
- an internal compartment;
- a kit for electric domestic appliances of the type previously described.

The packaged electric domestic appliance contains this kit inside the internal compartment, in order that the kit carries out a deodorising and/or aromatising and/or sanitising action in the compartment.

The electric domestic appliance according to this invention and containing the above-described kit can comprise:
i) a refrigerator, or a freezer or a fridge-freezer, comprising a refrigerator compartment area and/or a freezer compartment in which foodstuffs to be cooled and/or frozen are placed (in this case, the kit according to this invention is positioned in the compartment, for example on the lower wall of the compartment or on one of the shelves inside the compartment);
ii) a washing machine, a tumble-dryer or a combined washer-dryer, comprising a drum, which rotates around its own axis, in which items of clothing to be washed and/or dried are placed (in this case, the kit according to the invention is positioned in the drum);
iii) a dishwasher, comprising an internal compartment in which dishes to be washed are placed (in this case, the kit according to this invention is positioned in the internal compartment).

The concentration of scented essences in the air of the internal compartment of the appliance is preferably sufficient to exceed the concentration of monomers released by the plastic (one of the sources of unpleasant odours) and the chemical composition of the scented essences is preferably such that the human sense of smell is more sensitive to this composition.

This description clearly shows that to produce a kit for electric domestic appliances, in particular for refrigeration appliances or washing and/or drying appliances, according to this invention, an innovative procedure can be used, this procedure also being the subject of this invention.

This procedure intends to provide a kit for electric domestic appliances of the type described above. The procedure foresees the following steps:
i) adding an additional component to the basic plastic material in order to obtain a mixture of the basic plastic material and the additional component;
ii) modelling of this mixture to obtain a sheet. This step can be carried out by moulding or extrusion of the mixture. Advantageously, a film is obtained from the modelling step, in particular a film is obtained during the modelling step. The thickness of the film is preferably between 0.01 mm and 0.5 mm, even more preferably between 0.1 mm and 0.3 mm, advantageously 0.2 mm. The modelling step of this sheet comprises the step of cutting the film to obtain a sheet. Between the modelling step (in particular the modelling step of the film) and the cutting step, a winding step can be carried out, making the film easy to transport, for example winding it to form a reel;
iii) positioning the paper documentation on the sheet;
iv) forming a holder from the sheet, the paper documentation remaining housed in this holder.

Step iv) of forming a holder from the sheet can be carried out by folding the sheet and subsequently sealing the edges of the sheet in order to form the holder which holds the manual.

The additional component mentioned above comprises a scented essence and/or a component with a sanitising action. The scented essence preferably constitutes from 1% to 20% of the weight of basic plastic material-scented essence mixture; even more preferably from 8% to 12%, advantageously around 10%. An example of an additional component with a sanitising action is silver ions.

The production of the holder is similar to the production of the bags currently used to hold instruction manuals. One difference lies in the fact that in the loading hopper (which contains the basic material, typically in granular form, used to obtain the plastic film from which the holder is then formed) the basic plastic material (for example polyethylene) is mixed with the desired quantity of additional component (for example an essence with a strawberry aroma, such as 1-pentyl acetate or the component with a sanitising action), making the mixture of the basic plastic material and the additional component as homogeneous as possible so that the additional component is uniformly distributed on the sheet used to form the holder.

The adding step can also consist of loading the hopper with the basic plastic material already premixed with the additional component. It is obvious that the holder according to this invention does not require any additional processing with respect to bags known to the prior art, so the sole increase in cost, which is extremely limited to the point of being insignificant, consists of the difference in cost between the additional component and the amount of basic plastic material that may have been removed and replaced by the additional component.

The step of homogenising the mixture (in solid or liquid state) is advantageously foreseen between the adding step and the modelling step.

The film is then cut to obtain a sheet of plastic material and scented essence. The paper documentation (instruction manual and/or other documentation accompanying the manual, such as for example identification labels, gadgets, etc.) is placed on the sheet. The sheet is then folded (preferably along a symmetric axis of the sheet) so that the manual is positioned between two opposite flaps of the folded sheet. The creation of the kit according to this invention is then completed with a closing operation (for example sealing which, by joining the overlapping edges of the folded sheet, creates the holder and encloses the manual inside the holder).

The holder according to this invention can be closed on three sides, or on two sides parallel to each other and adjacent to the folded side. In this second case, the closure of the holder can be completed by applying adhesive tape or reversible opening or closing means, such as for example clamps or closure strips, on the free edges.

As an alternative to the adding step, a kit can be obtained with the following steps:
- applying on the inner layer of the holder the outer surface layer chemically different from the inner layer, this outer surface layer comprising a component that emits the particles;
- inserting the paper documentation in this holder.

The particles can have a deodorising and/or aromatising and/or sanitising action. The application of the outer surface layer on the inner layer of the holder preferably comprises a step of painting the inner layer with a paint that contains the component that emits the particles.

The particle-emitting component advantageously comprises a scented essence and/or a component with a sanitising action.

The insertion of the paper documentation in the holder can take place before or after the application of the outer surface layer. This alternative method of forming the holder is, however, less advantageous than the method previously described as it adds a further processing step, which means an increase in cost and a more complex production process of the kit.

Once the kit has been formed, it is positioned in the internal compartment of the electric domestic appliance according to this invention in exactly the same way as currently foreseen for the insertion of bags containing instruction manuals in electric domestic appliances. This makes it possible to scent the internal compartment without any additional operation with respect to those currently performed in the production of the electric domestic appliance. The kit can be left loose in the internal compartment of the appliance, or fixed for example by means of adhesive tape: it will in any case be extracted by the user before operating the appliance, as commonly occurs nowadays for the instruction booklets for known electric domestic appliances.

This invention provides numerous advantages.

Firstly, the kit neutralises, or considerably attenuates, the unpleasant odour released from the internal compartment of electric domestic appliances (such as refrigeration appliances or washing appliances), when, after removing the packaging, the door of the internal compartment is opened. It should be remembered that, in addition to being somewhat disagreeable, this unpleasant odour can condition the commercial success of an electric domestic appliance, the unpleasant odour being traditionally associated with an impression of poor quality.

Secondly, the kit easily adapts to different types of electric domestic appliances: for example, once it has been produced, it can be placed inside a refrigeration appliance or a washing and/or drying appliance, since it in any case has an effective scenting action countering unpleasant odours, whatever their cause. The only condition whereby a kit according to this invention should be placed in a refrigerator, rather than a washing machine, a dishwasher or a tumble-dryer, depends on the type of instructions in the manual.

Thirdly, the kit (unlike alternative methods for removing unpleasant odours, such as adding a component to the plastic used for the inner walls of the refrigerator or freezer) is removed before the appliance is put into operation: in the case of a refrigeration appliance, this has the fundamental advantage of there not being any scent in the appliance when it is functioning, as this could alter or cover the aromas of the foodstuffs contained in the appliance (for example, if strawberry essence is added to the plastic of the inner walls of a refrigerator, it will present the disadvantage of smelling of strawberry even when foodstuffs other than strawberries, such as cuts of meat, etc., are stored in it).

Fourthly, the kit (unlike all the alternative methods for removing unpleasant odours) does not affect the production cycle of the electric domestic appliance, since in the most economical form of this invention, in which the scented essence is applied to the holder by mixing the additional component with the basic material, no extra processing step is added with respect to those normally carried out.

Fifthly, the kit does not require the user to perform any additional operation with respect to what would normally be done before operating an electric domestic appliance for the first time.

Sixthly, the kit has the advantage of comprising a holder distinguished by a limited thickness: this means that a significant part of the scented essence comes into contact with the air in the internal compartment of the appliance and is therefore effective.

The invention described is susceptible to industrial application and may be modified and adapted in several ways without thereby departing from the scope of the inventive concept. Moreover, all the details of the invention may be substituted by technically equivalent elements. The invention is in fact described with reference to particular embodiments, but numerous variations are clearly possible according to the knowledge of experts in the sector and such variations lie within the framework of protection defined by the appended claims.

## Claims

1. A packaged electric domestic appliance comprising:
- an internal compartment accessible to the user and designed to house the products or objects for which the appliance is designed;
- a kit for said electric domestic appliance comprising a first component consisting of paper documentation relative to the appliance and a second component consisting of a holder that defines an internal cavity;
**characterised in that**:
i) the paper documentation is contained in the internal cavity;
ii) at least one of these first and second components emits particles able to counter the unpleasant odours of the surrounding outer environment;
iii) the packaged electric domestic appliance contains the kit in its internal compartment.

2. An appliance according to claim 1, **characterised in that** the holder emits particles able to counter the unpleasant odours of the surrounding outer environment.

3. An appliance according to claim 1 or 2, **characterised in that** the particles have a deodorising action.

4. An appliance according to claim 1, 2 or 3, **characterised in that** the particles have an aromatising action.

5. An appliance according to claim 3 or 4, **characterised in that** the material of the holder comprises a scented essence that emits these particles with an aromatising and/or deodorising action.

6. An appliance according to claim 5, **characterised in that** the scented essence constitutes from 1% to 20% of the weight of the holder.

7. An appliance according to claim 5 or 6, **characterised in that** the scented essence constitutes from 8% to 12% of the weight of the holder, preferably 10%.

8. An appliance according to any of the foregoing claims from 5 to 7, **characterised in that** the scented essence is uniformly distributed in the holder.

9. An appliance according to any of the foregoing claims from 1 to 7, **characterised in that** the holder comprises:
- an inner layer;
- an outer surface layer chemically different from the inner layer, the outer surface layer emitting the particles.

10. An appliance according to claim 9, **characterised in that** the inner layer and the outer surface layer adhere closely to each other.

11. An appliance according to any of the foregoing claims
**characterised in that** the particles have a sanitising action.

12. An appliance according to claim 11, **characterised in that** the particles comprise silver ions.

13. An appliance according to any of the foregoing claims
**characterised in that** the paper documentation comprises an instruction manual.

14. An appliance according to any of the foregoing claims
**characterised in that** at least part of the paper documentation emits the particles, the holder being permeable to these particles to allow them to pass through the holder from the inside to the outside.

15. An appliance according to any of the foregoing claims
**characterised in that** the thickness of the holder is between 0.01 mm and 0.5 mm.

16. An appliance according to any of the foregoing claims
**characterised in that** the thickness of the holder is between 0.1 mm and 0.3 mm, preferably 0.2 mm.

17. An appliance according to any of the foregoing claims
**characterised in that** the holder is flexible.

18. An appliance according to any of the foregoing claims
**characterised in that** the holder consists of a bag.

19. A procedure for the production of a kit for electric domestic appliances according to any of the claims from 1 to 18, comprising the steps of:
- adding an additional component to a basic plastic material in order to obtain a mixture of the basic plastic material and the additional component, the additional component comprising a scented essence and/or a component with a sanitising action;
- modelling of this mixture to obtain a sheet;
- positioning of paper documentation relative to the appliance on the sheet and
- forming a holder from the sheet, the paper documentation remaining housed in this holder.

20. A procedure according to claim 19, **characterised in that** the formation of the holder is carried out by folding the sheet and subsequently sealing the edges of the sheet.

21. A procedure for the production of a kit for electric domestic appliances, according to claim 9 or 10 and consequently the kit comprising a holder which in turn comprises an inner layer and an outer surface layer chemically different from the inner layer, the outer surface layer emitting the particles able to counter unpleasant odours of the surrounding outer environment, said holder defining an internal cavity, the procedure comprising the steps of:
- applying on the inner layer of the holder the outer surface layer comprising a component that emits the particles;
- inserting paper documentation in the internal cavity of this holder.

22. A procedure according to claim 21, **characterised in that** the application of the outer surface layer on the inner layer of the holder comprises a step of painting the inner layer with a paint that contains the component that emits the particles.

23. A procedure according to claim 21 or 22, **characterised in that** the component that emits the particles comprises a scented essence and/or a component with a sanitising action.

## Patentansprüche

1. Verpacktes elektrisches Haushaltsgerät, enthaltend:
- ein internes Fach, zugänglich für den Benutzer und dazu bestimmt, die Produkte oder Gegenstände aufzunehmen, für welche das Gerät vorgesehen ist;
- ein Set für das genannte elektrische Haushaltsgerät, enthaltend eine erste Komponente, bestehend aus der papiernen Dokumentation betreffend das Haushaltsgerät, und eine zweite Komponente, bestehend aus einem Behälter, der einen internen Hohlraum beschreibt;
**dadurch gekennzeichnet, dass**
i) die das Haushaltsgerät betreffende papierne Dokumentation in dem internen Hohlraum enthalten ist;
ii) wenigstens eine dieser ersten und zweiten Komponenten Partikel freigibt, die in der Lage sind, unangenehmen Gerüchen der umgebenden äusseren Umhüllung entgegen zu wirken;
iii) das verpackte elektrische Haushaltsgerät das Set in seinem internen Fach enthält.

2. Haushaltsgerät nach Patentanspruch 1, **dadurch gekennzeichnet, dass** der Behälter Partikel freigibt, die in der Lage sind, unangenehmen Gerüchen der umgebenden äusseren Umhüllung entgegen zu wirken.

3. Haushaltsgerät nach Patentanspruch 1 oder 2, **dadurch gekennzeichnet, dass** die Partikel eine desodorierende Wirkung haben.

4. Haushaltsgerät nach Patentanspruch 1, 2 oder 3, **dadurch gekennzeichnet, dass** die Partikel eine aromatisierende Wirkung haben.

5. Haushaltsgerät nach Patentanspruch 3 oder 4, **dadurch gekennzeichnet, dass** das Material des Behälters eine wohlriechende Essenz enthält, welche diese Partikel mit einer aromatisierenden und/oder desodorierenden Wirkung freigibt.

6. Haushaltsgerät nach Patentanspruch 5, **dadurch gekennzeichnet, dass** die wohlriechende Essenz von 1% bis 20% des Behältergewichtes bildet.

7. Haushaltsgerät nach Patentanspruch 5 oder 6, **dadurch gekennzeichnet, dass** die wohlriechende Essenz von 8% bis 12% des Behältergewichtes bildet, vorzugsweise 10%.

8. Haushaltsgerät nach einem jeden der vorstehenden Patentansprüche von 5 bis 7, **dadurch gekennzeichnet, dass** die wohlriechende Essenz gleichmässig in dem Behälter verteilt ist.

9. Haushaltsgerät nach einem jeden der vorstehenden Patentansprüche von 1 bis 7, **dadurch gekennzeichnet, dass** der Behälter wie folgt enthält:
- eine innere Schicht;
- eine äussere Oberflächenschicht, chemisch unterschiedlich von der inneren Schicht, wobei die äussere Oberflächenschicht die Partikel freigibt.

10. Haushaltsgerät nach Patentanspruch 9, **dadurch gekennzeichnet, dass** die innere Schicht und die äussere Oberflächenschicht dicht aneinander anhaften.

11. Haushaltsgerät nach einem jeden der vorstehenden Patentansprüche, **dadurch gekennzeichnet, dass** die Partikel eine sterilisierende Wirkung haben.

12. Haushaltsgerät nach Patentanspruch 11, **dadurch gekennzeichnet, dass** die Partikel Silberionen enthalten.

13. Haushaltsgerät nach einem jeden der vorstehenden Patentansprüche, **dadurch gekennzeichnet, dass** die papierne Dokumentation ein Bedienungshandbuch enthält.

14. Haushaltsgerät nach einem jeden der vorstehenden Patentansprüche, **dadurch gekennzeichnet, dass** wenigstens ein Teil der papiernen Dokumentation die Partikel freigibt, wobei der Behälter für diese Partikel durchlässig ist, um es ihnen zu ermöglichen, durch den Behälter von innen nach aussen zu gelangen.

15. Haushaltsgerät nach einem jeden der vorstehenden Patentansprüche, **dadurch gekennzeichnet, dass** die Dicke des Behälters zwischen 0,01 mm und 0,5 mm beträgt.

16. Haushaltsgerät nach einem jeden der vorstehenden Patentansprüche, **dadurch gekennzeichnet, dass** die Dicke des Behälters zwischen 0,1 mm und 0,3 mm beträgt, vorzugsweise 0,2 mm.

17. Haushaltsgerät nach einem jeden der vorstehenden Patentansprüche, **dadurch gekennzeichnet, dass** der Behälter flexibel ist.

18. Haushaltsgerät nach einem jeden der vorstehenden Patentansprüche, **dadurch gekennzeichnet, dass** der Behälter aus einer Tasche besteht.

19. Verfahren zur Herstellung eines Sets für elektrische Haushaltsgeräte nach einem jeden der Patentansprüche von 1 bis 18, enthaltend die folgenden Schritte:
- Zugabe einer zusätzlichen Komponente zu einem Kunststoffgrundmaterial, um eine Mischung aus dem Kunst-stoffgrundmaterial und der zusätzlichen Komponente zu erhalten, wobei die zusätzliche Komponente eine wohlriechende Essenz und/oder einen Bestandteil mit einer sterilisierenden Wirkung enthält;
- Verarbeiten dieser Mischung, um eine Folie zu erhalten;
- Positionieren der das Haushaltsgerät betreffenden papiernen Dokumentation auf der Folie; und
- Formen eines Behälters aus der Folie, wobei die papierne Dokumentation in diesem Behälter eingeschlossen bleibt.

20. Verfahren nach Patentanspruch 19, **dadurch gekennzeichnet, dass** das Formen des Behälters ausgeführt wird durch Falten der Folie und anschliessendes Versiegeln der Ränder der Folie.

21. Verfahren zur Herstellung eines Sets für elektrische Haushaltsgeräte nach Patentanspruch 9 oder 10 und folglich das Set, enthaltend einen Behälter, welcher wiederum eine innere Schicht und eine äussere Oberflächenschicht enthält, chemisch unterschiedlich von der inneren Schicht, wobei die äussere Oberflächenschicht die Partikel freigibt, die in der Lage sind, unangenehmen Gerüchen der umgebenden äusseren Umhüllung entgegen zu wirken, wobei der Behälter einen internen Hohlraum beschreibt, und wobei das Verfahren die folgenden Schritte enthält:
- Aufbringen auf die innere Schicht des Behälters der äusseren Oberflächenschicht, enthaltend eine Komponente, welche die Partikel freigibt;
- Einsetzen der papiernen Dokumentation in den internen Hohlraum dieses Behälters.

22. Verfahren nach Patentanspruch 21, **dadurch gekennzeichnet, dass** das Aufbringen der äusseren Oberflächenschicht auf die innere Schicht des Behälters einen Schritt des Bestreichens der inneren Schicht mit einem Anstrich umfasst, der die Komponente enthält, welche die Partikel freigibt.

23. Verfahren nach Patentanspruch 21 oder 22, **dadurch gekennzeichnet, dass** die Komponente, welche die Partikel freigibt, eine wohlriechende Essenz enthält und/oder mit einer sterilisierenden Wirkung versehen ist.

## Revendications

1. Appareil ménager électrique emballé comprenant:
- un compartiment interne accessible à l'utilisateur et conçu pour accueillir les produits ou objets pour lesquels l'appareil est conçu;
- un kit pour ledit appareil ménager électrique comprenant un premier composant constitué par une documentation sur papier relative à l'appareil et un second composant constitué par une enveloppe qui définit une cavité interne;
**caractérisé en ce que**:
i) la documentation sur papier est contenue dans la cavité interne;
ii) au moins l'un de ces premier et second composants émet des particules en mesure de contraster les mauvaises odeurs de l'environnement proche externe;
iii) l'appareil ménager électrique emballé contient le kit dans son compartiment interne.

2. Appareil selon la revendication 1, **caractérisé en ce que** l'enveloppe émet des particules en mesure de contraster les mauvaises odeurs de l'environnement proche externe.

3. Appareil selon la revendication 1 ou 2, **caractérisé en ce que** les particules ont une action désodorisante.

4. Appareil selon la revendication 1, 2 ou 3,
**caractérisé en ce que** les particules ont une action aromatique.

5. Appareil selon la revendication 3 ou 4, **caractérisé en ce que** le matériau de l'enveloppe comprend une essence parfumée qui émet ces particules avec une action aromatique et/ou désodorisante.

6. Appareil selon la revendication 5, **caractérisé en ce que** l'essence parfumée constitue 1 à 20% du poids de l'enveloppe.

7. Appareil selon la revendication 5 ou 6, **caractérisé en ce que** l'essence parfumée constitue de 8 à 12% du poids de l'enveloppe, préférablement 10%.

8. Appareil selon l'une quelconque des revendications 5 à 7, **caractérisé en ce que** l'essence parfumée est uniformément distribuée dans l'enveloppe.

9. Appareil selon l'une quelconque des revendications 1 à 7, **caractérisé en ce que** l'enveloppe comprend:
- une couche interne;
- une couche externe superficielle chimiquement différente de la couche interne, la couche externe superficielle émettant les particules.

10. Appareil selon la revendication 9, **caractérisé en ce que** la couche interne et la couche externe superficielle adhèrent entre elles.

11. Appareil selon l'une quelconque des revendications précédentes, **caractérisé en ce que** les particules ont une action désinfectante.

12. Appareil selon la revendication 11, **caractérisé en ce que** les particules comprennent des ions d'argent.

13. Appareil selon l'une quelconque des revendications précédentes, **caractérisé en ce que** la documentation sur papier comprend un manuel d'instructions.

14. Appareil selon l'une quelconque des revendications précédentes, **caractérisé en ce qu'**au moins une partie de la documentation sur papier émet des particules, l'enveloppe étant perméable à ces particules pour leur permettre de passer au travers de l'enveloppe de l'intérieur à l'extérieur.

15. Appareil selon l'une quelconque des revendications précédentes, **caractérisé en ce que** l'épaisseur de l'enveloppe est comprise entre 0,01 et 0,50 mm.

16. Appareil selon l'une quelconque des revendications précédentes, **caractérisé en ce que** l'épaisseur de l'enveloppe est comprise entre 0,1 et 0,3 mm, préférablement 0,2 mm.

17. Appareil selon l'une quelconque des revendications précédentes, **caractérisé en ce que** l'enveloppe est flexible.

18. Appareil selon l'une quelconque des revendications précédentes, **caractérisé en ce que** l'enveloppe consiste en un sac.

19. Procédure pour la production d'un kit pour appareils ménagers électriques selon l'une des revendications 1 à 18, comprenant les phases de:
- ajout d'un composant additionnel à un matériau plastique de base de manière à obtenir un mélange entre la matériau plastique de base et le composant additionnel, le composant additionnel comprenant une essence parfumée et/ou un composant ayant une action désinfectante;
- formage de ce mélange pour obtenir une feuille;
- positionnement de la documentation sur papier relative à l'appareil, et
- formage d'une enveloppe à partir de la feuille, la documentation sur papier restant logée dans cette enveloppe.

20. Procédure selon la revendication 19, **caractérisée en ce que** la formation de l'enveloppe est réalisée en pliant la feuille et successivement en soudant les bords de la feuille.

21. Procédure pour la production d'un kit pour des appareils ménagers électriques selon la revendication 9 ou 10, le kit comprenant en conséquence une enveloppe comprenant à son tour une couche interne et une couche externe superficielle chimiquement différente de la couche interne, la couche externe superficielle émettant des particules en mesure de contraster les mauvaises odeurs de l'environnement externe proche, ladite enveloppe définissant une cavité interne, la procédure comprenant les phases de:
- application sur là couche interne de l'enveloppe de la couche externe superficielle comprenant un composant qui émet les particules;
- insertion de la documentation sur papier dans la cavité interne de cette enveloppe.

22. Procédure selon la revendication 21, **caractérisée en ce que** l'application de la couche externe superficielle sur la couche interne de l'enveloppe comprend une phase de peinture de la couche interne avec une peinture contenant le composant qui émet les particules.

23. Procédure selon la revendication 21 ou 22,
**caractérisée en ce que** le composant qui émet les particules comprend une essence parfumée et/ou un composant ayant une action désinfectante.
